**Europäisches Patentamt**

**European Patent Office**

**Office européen des brevets**

(11) Veröffentlichungsnummer: **0 012 389**
**B1**

(12) **EUROPÄISCHE PATENTSCHRIFT**

(45) Veröffentlichungstag der Patentschrift:
**16.05.84**

(21) Anmeldenummer: **79104999.2**

(22) Anmeldetag: **07.12.79**

(51) Int. Cl.³: **A 61 M 5/14**

(54) **Infusionseinrichtung.**

(30) Priorität: **08.12.78 DE 2853200**

(43) Veröffentlichungstag der Anmeldung:
**25.06.80 Patentblatt 80/13**

(45) Bekanntmachung des Hinweises auf die Patenterteilung:
**16.05.84 Patentblatt 84/20**

(84) Benannte Vertragsstaaten:
**AT BE CH FR GB IT NL SE**

(56) Entgegenhaltungen:
**DE - A - 2 543 185**
**FR - A - 2 357 260**

(73) Patentinhaber: **Hirschmann, Josef, Fliederstrasse 2a,**
**D-8021 Neuried (DE)**

(72) Erfinder: **Hirschmann, Josef, Fliederstrasse 2a,**
**D-8021 Neuried (DE)**
Erfinder: **Iwatschenko, Peter, D-8524 Neunkirchen (DE)**

(74) Vertreter: **Wallach, Curt, Dipl.-Ing. et al, Postfach 920,**
**D-8000 München 33 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Infusionseinrichtung

Die Erfindung bezieht sich auf eine nach dem Schwerkraftprinzip arbeitende Infusionseinrichtung mit einem gegenüber der Infusionsstelle erhöht aufgehängten Lösungsmittelbehälter, an den eine Tropfenkammer angeschlossen ist, die über einen Infusionsschlauch mit einer Infusionsnadel verbunden ist, mit einer Schlauchklemme zur Veränderung des Querschnittes des Schlauches und mit einer Druckerhöhungseinrichtung zur Erhöhung des Druckes in dem Schlauchabschnitt zwischen Druckerhöhungseinrichtung und Infusionsnadel.

Bei der Schwerkraftinfusion wird der Druck für die zu infundierende Lösung durch das Eigengewicht der Lösung erzeugt. Dies erfolgt in der Praxis durch gegenüber der Infusionsstelle mit der Infusionskanüle erhöhtes Aufhängen des Lösungsmittelbehälters. Der durch die Schwerkraft erzeugte oder erhöhte Druck von ca. 0,15 bar = 1500 mm Wassersäule überwindet den Gegendruck des Körpers von ca. $8 \cdot 10^{-3}$ bar = 80 mm Wassersäule und infundiert die Flüssigkeit. Die Höhe des Druckes ist durch die Höhe der Aufhängung des Lösungsmittelbehälters gegeben.

Geht die Flüssigkeit in dem Lösungsmittelbehälter zu Ende und sinkt der Druck in der Flüssigkeit auf den Gegendruck des Körpers ab, so hört die Infusion auf. Eine Flüssigkeitsäule entsprechend dem Körpergegendruck bleibt stehen, so daß sichergestellt ist, daß keine Luft infundiert wird.

Die Infusionsgeschwindigkeit wird im einfachsten Fall mit Hilfe einer von Hand einstellbaren Schlauchklemme dosiert. Es sind weiterhin Regeleinrichtungen bekannt (DE-A-2 733 702), bei denen eine Regelschaltung die Infusionsgeschwindigkeit in Abhängigkeit von Ausgangssignalen einer Tropfenkammer durch periodisches Öffnen und Schließen einer Schlauchklemme steuert.

Bedingt durch das Prinzip der Erzeugung des für die Infusion erforderlichen Druckes mittels Schwerkraft kann bei derartigen Infusionseinrichtungen kein unzulässig hoher Druck entstehen und andererseits sinkt der Druck automatisch ab, wenn die Infusionslösung zu Ende geht und Luft in den zur Infusionsnadel führenden Schlauch gelangt.

Für spezielle Anwendungen, z. B. bei Infusionen in die dünnen Blutgefäße und bei Kindern ist jedoch eine Infusion mit erhöhtem Druck erforderlich. Zu diesem Zweck ist es bereits bekannt (DE-A-2 543 185), peristaltische oder andere Pumpen zu verwenden. Derartige Pumpeinrichtungen ergeben jedoch die Gefahr eines unzulässig hohen Druckanstiegs, beispielsweise bei einem Abklemmen des Schlauches zwischen Pumpeinrichtungen und Infusionsnadel und weiterhin ist bei derartigen Pumpeinrichtungen nicht sichergestellt, daß keine Luft zur Infusionsnadel gefördert wird.

Der Erfindung liegt die Aufgabe zugrunde, eine Infusionseinrichtung der eingangs genannten Art zu schaffen, bei der es möglich ist, den Infusionsdruck zu erhöhen, ohne daß die Gefahr einer Infusion von Luft besteht.

Diese Aufgabe wird durch die im Patentanspruch 1 angegebene Erfindung gelöst.

Weitere vorteilhafte Ausgestaltungen und Weiterbildungen der Erfindung ergeben sich aus den Unteransprüchen.

Durch die erfindungsgemäße Ausgestaltung der Infusionseinrichtung ist es möglich, den Infusionsdruck zu erhöhen, ohne daß die Gefahr der Infusion von Luft besteht, da in dem Infusionsschlauch lediglich periodische Druckschwankungen erzeugt werden, die sich bei mit Luft gefülltem Schlauch ohne weiteres ausgleichen können, so daß bei Auftreten einer Luftsäule zwischen Infusionsstelle und Preßeinrichtung immer noch eine ausreichende Flüssigkeitssäule vor der Infusionsstelle verbleibt.

Eine vorteilhafte Anwendung der erfindungsgemäßen Infusionseinrichtung ergibt sich in Verbindung mit Regeleinrichtungen, die auf eine gewünschte Infusionsgeschwindigkeit einstellbar sind und die in Abhängigkeit von der Anzahl der durch die Tropfenkammer fallenden Tropfen eine Schlauchklemme mehr oder weniger öffnen. Wenn bei Verwendung derartiger geregelter Infusionseinrichtungen eine Verstopfung der Infusionsnadel oder eine Verengung ihres wirksamen Querschnittes auftritt, so regelt die Regeleinrichtung die Schlauchklemme auf die größte Öffnungsweite ein und stellt dann fest, daß auch in dieser Stellung der Schlauchklemme kein Tropfen fällt. Bei Erreichen dieses einen Alarmzustand darstellenden Zustandes kann die Regeleinrichtung so ausgebildet sein, daß sie die Schlauchklemme völlig verschließt und die Steuereinrichtung der Preßeinrichtung ansteuert, so daß sie Druckschwankungen in der Flüssigkeitssäule zwischen der Schlauchklemme und der Infusionsnadel erzeugt, die in den meisten Fällen Verstopfungen oder Verengungen des Auslaßquerschnittes beseitigen. Die Druckerzeugung kann hierbei vorzugsweise in mehreren, beispielsweise drei Stufen erfolgen. Der Schlauchabschnitt wird dabei jeweils unterschiedlich weit zusammengedrückt, wobei jedoch auch bei voller Druckerzeugung ein Flüssigkeitskanal zum Druckausgleich bestehen bleibt. Diese drei Stufen der Zusammendrükkung können nacheinander angesteuert werden, wobei nach jeder Stufe die Schlauchklemme wieder geöffnet wird und ermittelt wird, ob in angemessener Zeit ein Tropfen in der Tropfenkammer fällt. Die Regeleinrichtung schaltet hierbei die Preßeinrichtung bei negativem Überprüfungsergebnis auf die nächsthöhere Stufe der Druckerzeugung, während bei positivem Ergebnis auf die nächstniedrige Stufe zurückgeschaltet werden kann.

Daher geht die gesamte Infusionseinrichtung bei kurzzeitigen Verschlüssen des Infusionskanals anschließend wieder automatisch in den Schwerkraftbetrieb zurück, während sie dauernd zwischen den verschiedenen Druckerzeugungsstufen pendelt, wenn dauernd eine Druckunterstützung zur Erzielung der gewünschten Infusionsgeschwindigkeit erforderlich ist.

Die Erfindung wird im folgenden anhand von in der Zeichnung dargestellten Ausführungsbeispielen noch näher erläutert. In der Zeichnung zeigt

Fig. 1 eine bekannte Ausführungsform einer nach dem Schwerkraftprinzip arbeitenden Infusionseinrichtung,

Fig. 2 eine Ausführungsform der erfindungsgemäßen Infusionseinrichtung,

Fig. 3 eine Ausführungsform der Preßeinrichtung zur Erhöhung des Druckes,

Fig. 4 eine weitere Ausführungsform einer Preßeinrichtung zur Erhöhung des Druckes,

Fig. 5 eine dritte Ausführungsform einer Preßeinrichtung zur Erhöhung des Druckes.

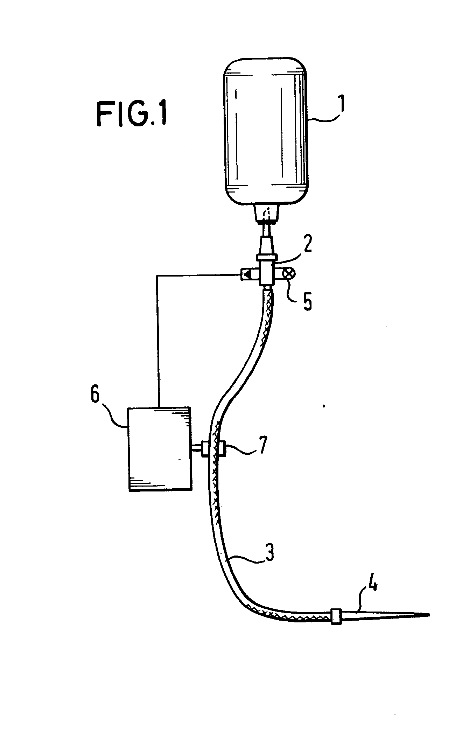

In Fig. 1 ist eine bekannte nach dem Schwerkraftprinzip arbeitende Infusionseinrichtung dargestellt. Diese Infusionseinrichtung weist einen Lösungsmittelbehälter 1, eine Tropfenkammer 2, einen Infusionsschlauch 3 und eine Infusionsnadel 4 auf. Weiterhin ist diese Infusionseinrichtung mit einer Regeleinrichtung zur Regelung der Infusionsgeschwindigkeit versehen, die einen Tropfensensor 5 und eine über eine elektronische Regeleinrichtung 6 gesteuerte Schlauchklemme 7 einschließt.

Der Querschnitt des Schlauches 3 im Bereich der Schlauchklemme 7 wird in Abhängigkeit von den durch die Tropfenkammer 2 fallenden Tropfen durch die Regeleinrichtung 6 auf einen gewünschten Wert eingeregelt.

Bei einfacheren derartigen Infusionseinrichtungen wird die Schlauchklemme 7 manuell auf einem empirisch oder aufgrund von Erfahrung gewonnenen Wert eingestellt.

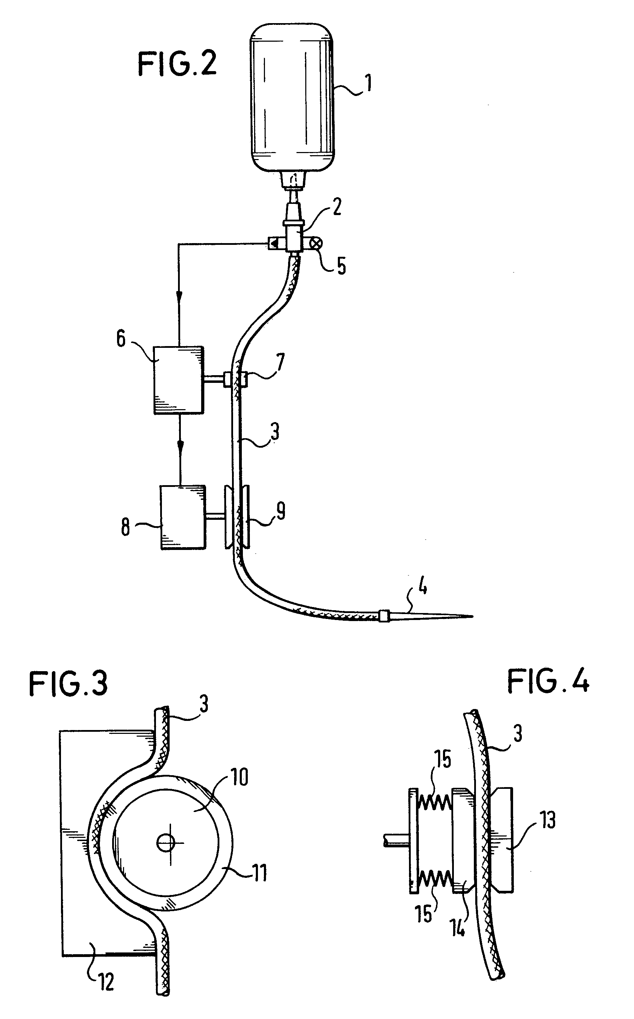

Bei vorübergehendem stärkerem Gegendruck an der Infusionsstelle oder bei einer Infusion in sehr enge Blutgefäße kann jedoch das Fallen des Tropfens durch die Tropfenkammer 2 verhindert werden. Um diesen Nachteil zu beseitigen, ist bei der in Fig. 2 dargestellten Ausführungsform eine Preßeinrichtung 9 vorgesehen, die eine Zusammenpressung des Schlauches zwischen der Schlauchklemme 7 und der Infusionsstelle 4 ermöglicht, so daß in diesem Bereich des Schlauches eine Druckerhöhung erzielt wird.

Im Normalfall arbeitet die Infusionseinrichtung mit der Druckerzeugung durch die Schwerkraft. Ist dieser Druck jedoch nicht ausreichend, so kann die Preßeinrichtung 9 mit Hilfe der Steuereinrichtung 8 entweder unter manueller Steuerung oder unter der Steuerung durch eine übliche Regeleinrichtung 6 einzelne oder periodische Druckschwankungen in dem Bereich des Schlauches zwischen der Schlauchklemme 7 und der Infusionsnadel 4 erzeugen. Zu diesem Zweck kann in dem Bereich des Schlauches 3 zwischen der Tropfenkammer 2 und der Preßeinrichtung 9 entweder ein Rückschlagventil oder eine den Querschnitt des Schlauches verengende Einrichtung vorgesehen sein oder die durch die Regeleinrichtung 6 betätigte Schlauchklemme 7 kann zu diesem Zweck herangezogen werden. Die Preßeinrichtung 9 kann für einen unabhängigen Betrieb ohne eine Regeleinrichtung 6 von der Steuereinrichtung 8 periodisch zusammengedrückt werden, so daß sich eine pulsierende Druckschwingung der Flüssigkeitssäule in dem Infusionsschlauch ergibt, wodurch die Flüssigkeit aus dem Lösungsmittelbehälter zum Patienten gefördert wird.

Bei Verwendung der Regeleinrichtung 6 stellt diese mit Hilfe eines Tropfensensors 5 das Fallen oder Nichtfallen eines Tropfens in der Tropfenkammer fest. In Abhängigkeit von der gewünschten Anzahl der Tropfen pro Zeiteinheit und damit der Infusionsgeschwindigkeit wird die Schlauchklemme 7 durch diese Regeleinrichtung mehr oder weniger geöffnet. Wenn aufgrund einer Verstopfung oder einer Verengung des Infusionskanals kein Tropfen oder zu wenige Tropfen pro Zeiteinheit durch die Tropfenkammer fallen, so steuert die Regeleinrichtung 6 die Steuereinrichtung 8 an, wodurch der in der Preßeinrichtung 9 befindliche Abschnitt des Schlauches zusammengedrückt wird und die hierdurch hervorgerufene Druckerhöhung den Austritt der Infusionsflüssigkeit durch die Infusionsnadel 4 bewirkt. Vor der Zusammenpressung des Schlauches wirkt die Regeleinrichtung 6 auf die Schlauchklemme 7 so ein, daß der Querschnitt des Schlauches im Bereich dieser Schlauchklemme 7 zumindest stark verringert, wenn nicht vollkommen geschlossen wird.

Solange Flüssigkeit im Schlauch 3 ist, entsteht durch das Pressen des Schlauchabschnittes in der Preßeinrichtung 9 Überdruck im System, der durch die Elastizität des Schlauches jedoch begrenzt ist. Gelangt Luft in das System, so läßt sich diese komprimieren und der Druck steigt nur unwesentlich an. Durch die Dimensionierung der Preßeinrichtung, insbesondere die Länge des durch die Preßeinrichtung 9 zusammengepreßten Abschnittes des Schlauches 3 zwischen der Schlauchklemme 7 und der Infusionsnadel 4 wird der maximale Druck bestimmt, der entsteht, wenn Luft in das System gelangt. Der Druck kann damit so begrenzt werden, daß er sicher unter dem Körpergegendruck bleibt und Luftinfusion verhindert wird.

Der maximal im Schlauch 3 entstehende Druck wird entweder durch die Elastizität des Schlauches oder durch Begrenzung der Kraft auf die Preßeinrichtung 9 begrenzt.

Die Preßeinrichtung 9 wird in Abhängigkeit von der Anzahl der durch die Tropfenkammer 2 fallenden Tropfen sowie der jeweiligen Stellung der Schlauchklemme 7 und der zu fördernden Infusionsmenge gesteuert.

Ausführungsbeispiele der Preßeinrichtungen werden im folgenden anhand der Fig. 3 bis 5 erläutert.

Die in Fig. 3 dargestellte Ausführungsform der Preßeinrichtung besteht aus einer im wesentlichen kreisförmig konkav geformten Auflageplatte 12, auf der der Abschnitt des Schlauches aufliegt, wobei eine Exzenterscheibe 10 über einen Gleitring 11 auf diesen Schlauchabschnitt drückt. Durch Einstellung des Abstandes zwischen der Platte 12 und der Achse des Exzenters 10 ist eine Einstellung der maximalen Druckerhöhung möglich, während die Drehzahl des Exzenters 10 die Häufigkeit der Druckschwankungen pro Zeiteinheit bestimmt. Bei kontinuierlicher Drehung des Exzenters lassen sich auf diese Weise periodische Druckschwankungen in der Flüssigkeitssäule erzielen.

Bei der in Fig. 4 dargestellten Ausführungsform der Preßeinrichtung ist der Schlauchabschnitt des Schlauches 3 zwischen zwei Platten 13, 14 angeordnet, von denen die mit 14 bezeichnete Platte über Federn 15 mit Hilfe einer Antriebseinrichtung gegen die Platte 13 gedrückt werden kann, um den Schlauch zusammenzudrücken. Der Antrieb kann in diesem Fall mit Hilfe eines Elektromagneten oder mit Hilfe eines Elektromotors erfolgen. Die Begrenzung der Kraft erfolgt durch die Federelemente 15 an den Druckbacken oder durch Begrenzung des Stromes des Antriebselementes. Eine weitere Begrenzung ergibt sich in der vorstehend beschriebenen Weise durch die Elastizität des Schlauches.

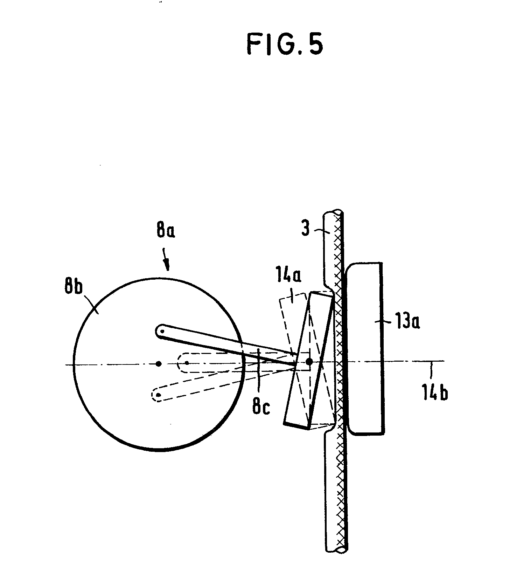

Fig. 5 zeigt eine dritte Ausführungsform der Preßeinrichtung, die ähnlich der Ausführungsform nach Fig. 4 ist, bei der jedoch eine Platte 14a eine Taumelbewegung mit Hilfe eines Mechanismus 8a ausführt, der einen Teil der Steuereinrichtung 8 nach Fig. 2 bildet. Dieser Mechanismus 8a schließt eine Kurbelscheibe 8b ein, die selektiv mit Hilfe von nicht dargestellten Betriebseinrichtungen in Drehung versetzt werden kann und die eine Kurbelstange 8c antreibt, die starr mit der Platte 14a an deren Mittelpunkt verbunden ist. Der Mittelpunkt der Platte 14a ist weiterhin entlang der gestrichelten geraden Linie 14b senkrecht zur Ebene der Vorderfläche der Platte 13a geführt, die mit dem Schlauch 3 in Berührung steht. Auf diese Weise führt die Platte 14a eine Taumelbewegung aus, bei der zu einem ersten Zeitpunkt lediglich die obere Hälfte der Platte mit dem Schlauch 3 in Berührung kommt, so daß dieser in kontinuierlich anwachsendem Ausmaß zusammengedrückt wird, bis die Platte 14a parallel zur Platte 13a angeordnet ist. Daraufhin bewegt sich die obere Hälfte der Platte 14a von dem Schlauch 3 fort, wobei lediglich die untere Hälfte der Platte 14a in Eingriff mit dem Schlauch 3 bleibt. Schließlich wird die Platte 14a vollständig von dem Schlauch 3 fortbewegt.

Durch die Taumelbewegung der Platte 14a wird ein sanftes und kontinuierliches Ansteigen des Druckes in dem Schlauch 3 erzielt. Der Mechanismus 8a kann selbstverständlich durch andere bekannte Mechanismen zur Erzielung der Taumelbewegung ersetzt werden.

Selbstverständlich sind auch weitere Ausgestaltungen der Preßeinrichtung denkbar, da es lediglich darauf ankommt, einen gewissen Abschnitt des Schlauches kurzzeitig oder periodisch zusammenzudrücken. Weiterhin ist es nicht erforderlich, daß der Schlauch 3 mit dem zusammengepreßten Abschnitt ein kontinuierliches durchgehendes Teil bildet, sondern dieser Abschnitt kann einerseits aus von dem übrigen Schlauch 3 abweichendem Material bestehen, wie z. B. aus Silikonkautschuk, und weiterhin kann dieser Schlauchabschnitt eine andere Querschnittsform oder eine andere Querschnittsfläche aufweisen, als der übrige Schlauch 3, und zwar in Abhängigkeit von der gewünschten Druckerhöhung. Weiterhin ist es möglich, den zusammengedrückten Schlauchabschnitt als getrennten Schlauchabschnitt herzustellen, der mit dem eigentlichen Infusionsschlauch 3 über eine Verzweigung verbunden ist.

Bei Verwendung der Preßeinrichtung 9 sowie der zugehörigen Steuereinrichtung 8 in Verbindung mit einem Infusionssystem mit einer Regeleinrichtung 6 gemäß Fig. 2 kann die Druckerzeugung in mehreren, beispielsweise drei Stufen erfolgen, bei denen der Schlauchabschnitt jeweils unterschiedlich weit zusammengedrückt wird. Auch bei voller Druckerzeugung bleibt ein Flüssigkeitskanal im Schlauchabschnitt an der gepreßten Stelle zum Druckausgleich.

Wenn die Regeleinrichtung 6 feststellt, daß Druckunterstützung erforderlich ist, so wird die Schlauchklemme 7 geschlossen. Anschließend wird die Preßeinrichtung 9 mit Hilfe der Steuereinrichtung 8 so angesteuert, daß sich die erste Stufe der Zusammenpressung des Schlauchabschnittes ergibt, worauf die Preßeinrichtung wieder in die Ausgangs- bzw. Ruhestellung zurückkehrt. Die Regeleinrichtung 6 prüft dann erneut durch Öffnen der Schlauchklemme 7, ob Infusionsflüssigkeit nachfließen kann. Ist dies nicht der Fall, wird die nächste Stufe der Zusammenpressung des Schlauchabschnittes angesteuert. Anschließend erfolgt erneut wieder die Prüfung, ob Flüssigkeit nachfließen kann.

Bei negativem Ergebnis der Prüfung durch die Regeleinrichtung, d. h. bei Nichtfeststellung eines ausreichenden Tropfenfalls in der Tropfenkammer bzw. fehlendem Ausgangssignal des Tropfensensors 5, wird jeweils die nächsthöhere Stufe der Preßeinrichtung 9 eingeschaltet. Bleibt die Prüfung auch bei der höchsten Druckstufe negativ, so gibt das Gerät Alarm.

Stellt die Regeleinrichtung 6 mit Hilfe des Tropfensensors 5 fest, daß Tropfen durch die Tropfenkammer 2 fallen und damit Lösung nachfließt, so schaltet sie bei der nachfolgenden Druckunterstützung der Preßeinrichtung 9 eine Stufe zurück. Bei kurzzeitigen Verschlüssen des Infusionskanals geht das System daher automatisch wieder in den reinen Schwerkraftbetrieb zurück. Bei andauernder erforderlicher Druckunterstützung pendelt das System jedoch dauernd zwischen den Stufen der Preßeinrichtung 9.

Die in den Fig. 3 bis 5 dargestellten Ausführungsformen der Preßeinrichtung stellen lediglich vorteilhafte mechanische Ausführungsformen dar.

Weitere vorteilhafte Ausführungsformen können durch Verwendung magnetostriktiver oder elektrostriktiver Elemente für die Platten 13, 14 bzw. die Platte 12 und den Gleitring 11 gebildet sein. In diesem Fall kann der elektromotorische oder elektromagnetische Antrieb entfallen, so daß keine mechanisch angetriebenen Elemente erforderlich sind.

**Patentansprüche**

1. Infusionseinrichtung mit einem gegenüber der Infusionsstelle erhöht aufgehängten Lösungsmittelbehälter (1), an den eine Tropfenkammer (2) angeschlossen ist, die über einen Infusionsschlauch (3) mit einer Infusionsnadel (4) verbunden ist, mit einer Schlauchklemme (7) zur Veränderung des Querschnitts des Schlauches (3) und mit einer Druckerhöhungseinrichtung (9) zur Erhöhung des Druckes in dem Schlauchabschnitt zwischen der Druckerhöhungseinrichtung und der Infusionsnadel (4), dadurch gekennzeichnet, daß bei einer nach dem Schwerkraftprinzip arbeitenden Infusionseinrichtung die Druckerhöhungseinrichtung (9) selektiv einschaltbar ist, wenn der sich aus der Schwerkraft ergebende Druck nicht ausreicht, um eine gewünschte Infusionsgeschwindigkeit zu erzielen, und daß die Druckerhöhungseinrichtung (9) durch eine einen elastisch ausgebildeten Abschnitt des Schlauches (3) steuerbar zusammendrückende Preßeinrichtung (9) gebildet ist, die Druckschwankungen in dem Schlauchabschnitt zwischen der Schlauchklemme (7) und der Infusionsnadel (4) hervorruft.

2. Infusionseinrichtung nach Anspruch 1, dadurch gekennzeichnet, daß die Preßeinrichtung (9) durch zwei Platten (13, 14) gebildet ist, zwischen denen der Abschnitt des Schlauches (3) hindurchgeführt ist und daß zumindest eine dieser Platten (13, 14) in Richtung auf die andere Platte antreibbar ist.

3. Infusionseinrichtung nach Anspruch 2, dadurch gekennzeichnet, daß die Platten (13, 14) planparallele Platten sind, von denen eine senkrecht zu ihrer Ebene in Richtung auf die andere Platte antreibbar ist.

4. Infusionseinrichtung nach Anspruch 3, dadurch gekennzeichnet, daß zumindest eine Platte (13a, 14a) eine Taumelbewegung relativ zur anderen Platte und in Richtung auf diese ausführt.

5. Infusionseinrichtung nach einem der Ansprüche 1 bis 4, dadurch gekennzeichnet, daß die Frequenz und/oder die Amplitude der Bewegung der zumindest einen Platte (13, 14) änderbar ist.

6. Infusionseinrichtung nach einem der Ansprüche 1 bis 5, dadurch gekennzeichnet, daß in die Preßeinrichtung (9) Platten (13, 14) mit unterschiedlicher, auf den Schlauchabschnitt einwirkender Fläche einsetzbar sind.

7. Infusionseinrichtung nach Anspruch 1, dadurch gekennzeichnet, daß die Preßeinrichtung (9) durch eine im wesentlichen kreisförmig konkav geformte Auflageplatte (12) gebildet ist, auf der der Schlauchabschnitt aufliegt, und daß entgegengesetzt zum Schlauch eine Exzentereinrichtung (10, 11) vorgesehen ist, die den Schlauchabschnitt gegen die Auflageplatte (12) zusammendrückt.

8. Infusionseinrichtung nach Anspruch 7, dadurch gekennzeichnet, daß die Exzentereinrichtung durch eine exzentrisch gelagerte Scheibe oder Rolle (10) gebildet ist, auf deren Außenumfang ein Gleitring (11) angeordnet ist, der gegenüber dem Exzenter (10) verdrehbar ist und den Schlauchabschnitt gegen die Auflageplatte (12) drückt.

9. Infusionseinrichtung nach den vorhergehenden Ansprüchen, dadurch gekennzeichnet, daß der Antrieb der zumindest einen Platte (14) bzw. des Exzenters (10) über einen Elektromagneten erfolgt.

10. Infusionseinrichtung nach einem der Ansprüche 1 bis 8, dadurch gekennzeichnet, daß der Antrieb der zumindest einen Platte (14) bzw. des Exzenters (10) über einen Elektromotor erfolgt.

11. Infusionseinrichtung nach einem der vorhergehenden Ansprüche, dadurch gekennzeichnet, daß der mit der Preßeinrichtung zusammenwirkende Schlauchabschnitt aus Silikonkautschuk besteht und ohne Querschnittsänderung mit einem PVC-Schlauch der Saug- und Druckseite verbunden ist.

12. Infusionseinrichtung nach einem der Ansprüche 1 bis 10, dadurch gekennzeichnet, daß der mit der Preßeinrichtung (9) zusammenwirkende Schlauchabschnitt eine andere Querschnittsform und/oder Querschnittsfläche aufweist als der übrige Teil des Infusionsschlauches.

13. Infusionseinrichtung nach einem der Ansprüche 1—10 und 12, dadurch gekennzeichnet, daß der mit der Preßeinrichtung (9) zusammenwirkende Schlauchabschnitt ein getrennter und mit dem eigentlichen Infusionsschlauch über ein Verzweigungsstück verbundener Schlauchabschnitt ist.

14. Infusionseinrichtung nach einem der vorhergehenden Ansprüche, dadurch gekennzeichnet, daß strömungsaufwärts von der Preßeinrichtung (9) ein Rückschlagventil und/oder eine den Strömungsquerschnitt des Schlauches verengende oder verschließende Einrichtung angeordnet ist.

15. Infusionseinrichtung nach einem der vorhergehenden Ansprüche, dadurch gekennzeichnet, daß ein die Infusionsgeschwindigkeit messender Meßfühler (2, 5) vorgesehen ist, dessen Ausgangssignal zur Ansteuerung der Antriebseinrichtungen der Druckerhöhungseinrichtung (9) dient.

16. Infusionseinrichtung nach einem der Ansprüche 1 bis 15, dadurch gekennzeichnet, daß die Preßeinrichtung zwischen einem durch eine Regeleinrichtung (6) zur Regelung der Infusionsgeschwindigkeit gesteuerten Absperrglied (7) und der Infusionsstelle angeordnet ist.

17. Infusionseinrichtung nach einem der vorhergehenden Ansprüche, dadurch gekennzeichnet, daß die Erzeugung der Druckschwankungen periodisch erfolgt.

**Claims**

1. Infusion apparatus comprising a solvent container (1) suspended above the point of infusion, with a droplet chamber (2) which is connected via an infusion hose (3) to an infusion needle (4) being attached to the solvent container; a hose clamp (7) for changing the cross-section of the hose (3); and a pressure increasing device (9) for increasing the pressure in the hose section between the pressure increasing device and the infusion needle (4), characterized in that, in an infusion apparatus which operates in accordance with the gravity principle, the pressure increasing device (9) is capable of being selectively switched on when the pressure resulting from gravity is insufficient to obtain a desired speed of infusion; and in that the pressure increasing device (9) is formed by a pressing device (9) which controllably presses together a resiliently constructed section of the hose (3) and which produces pressure variations in the hose section between the hose clamp (7) and the infusion needle (4).

2. Infusion apparatus in accordance with claim 1, characterized in that the pressing device (9) is formed by two Plates (13, 14) between which the section of the hose (3) is passed; and in that at least one of these plates (13, 14) can be driven in the direction towards the other plate.

3. Infusion apparatus in accordance with claim 2, characterized in that the plates (13, 14) are plane parallel plates of which the one can be driven at right angles to its plane towards the other.

4. Infusion apparatus in accordance with claim 3, characterized in that at least one plate (13a, 14a) executes a swashing motion relative to and in the direction towards the other plate.

5. Infusion apparatus in accordance with ohne of the claims 1 to 4, characeterised in that the frequency und/or the amplitude of the movement of at least one plate (13, 14) is variable.

6. Infusion apparatus in accordance with one of the claims 1 to 5, characterized in that plates (13, 14) with different surfaces which act on the hose section can be inserted into the pressing device (9).

7. Infusion apparatus in accordance with claim 1, characterized in that the pressing device (9) is formed by a support plate (12) of substantially circularly concave shape on which the hose section lies; and in that an eccentric device (10, 11) which presses the hose section against the support plate (12) ist provided on the opposite side of the hose.

8. Infusion apparatus in accordance with claim 7, characterized in that the eccentric device is formed by an eccentrically journalled disc or roller (10) on the outside of which there is arranged a bearing ring (11) which is rotatable relative to the eccentric (10) and which presses the hose section against the support plate (12).

9. Infusion apparatus in accordance with one of the preceding claims, characterized in that the drive of the at least one plate (14) or of the eccentric (10) takes place via an electromagnet.

10. Infusion apparatus in accordance with one of the claims 1 to 8, characterized in that the drive of the at least one plate (14) or of the eccentric (10) takes place via an electric motor.

11. Infusion apparatus in accordance with one of the preceding claims, characterized in that the hose section which cooperates with the pressing device consists of silicon rubber and is connected without a change in cross-section with a PVC hose on the suction and pressure sides.

12. Infusion apparatus in accordance with one of the claims 1 to 10, characterized in that the hose section which cooperates with the pressing device (9) has a different cross-sectional shape and/or cross-sectional area than the remaining part of the infusion hose.

13. Infusion apparatus in accordance with one of the claims 1 to 10 and 12, characterized in that the hose section which cooperates with the pressing device (9) is a separate hose section which is connected with the actual infusion hose via a branch piece.

14. Infusion apparatus in accordance with one of the preceding claims, characterized in that a non-return valve and/or a device which narrows or closes the flow cross-section of the hose is provided upstream of the pressing device (9).

15. Infusion apparatus in accordance with one of the preceding claims, characterized in that a measuring sensor (2, 5) which measures the speed of infusion is provided the output signal of which serves to control the drive means for the pressure increasing device (9).

16. Infusion apparatus in accordance with one of the claims 1 to 15, characterized in that the pressing device is arranged between a blocking member (7) controlled by a regulating device (6) to regulate the speed of infusion and the point of infusion.

17. Infusion apparatus in accordance with one of the preceding claims, characterized in that the generation of the pressure variations takes place periodically.

**Revendications**

1. Dispositif de perfusion comportant un récipient à solvant (1) suspendu en position surélevée par rapport à l'emplacement de la perfusion, récipient auquel est raccordée une chambre d'écoulement goutte à goutte (2), qui par un tuyau de perfusion (3) est reliée à une aiguille de perfusion (4), le dispositif comportant également une pince de serrage du tuyau (7) pour modifier la section transversale du tuyau (3) ainsi qu'un dispositif d'augmentation de la pression (9) pour augmenter la pression dans le tronçon de tuyau compris entre le dispositif d'augmentation de la pression et l'aiguille de perfusion (4), caractérisé en ce que dans le cas d'un dispositif de perfusion fonctionnant selon le principe de gravitation, le dispositif d'augmentation de la pression (9) est mis en action sélectivement lorsque la pression résultant de la force de pesanteur n'est pas suffisante pour l'obtention d'une vitesse de perfusion désirée et en ce que le dispositif d'augmentation de la pression (9) est constitué par un dispositif de presse (9) pouvant être commandé pour comprimer une section élastique du tuyau (3), ce dispositif provoquant des fluctuations de pression dans le tronçon de tuyau compris entre la pince de serrage (7) et l'aiguille de perfusion (4).

2. Dispositif de perfusion selon la revendication 1, caractérisé en ce que le dispositif de presse (9) se compose de deux plateaux (13, 14) entre lesquels le tronçon de tuyau (3) est intercalé et en ce qu'au moins un de ces plateaux (13, 14) peut être mû vers l'autre plateau.

3. Dispositif de perfusion selon la revendication 2, caractérisé en ce que les plateaux (13, 14) sont plans parallèles entre eux et l'un d'entre eux peut être mû perpendiculairement à son plan vers l'autre plateau.

4. Dispositif de perfusion selon la revendication 3, caractérisé en ce qu'au moins un plateau (13a, 14a) décrit un mouvement de bascule par rapport à l'autre plateau et vers celui-ci.

5. Dispositif de perfusion selon l'une des revendications 1 à 4, caractérisé en ce que la fréquence et/ou l'amplitude du mouvement d'au moins un plateau (13, 14) peuvent être modifiées.

6. Dispositif de perfusion selon l'une des revendications 1 à 5, caractérisé en ce que le dispositif de presse (9) peut mettre en oeuvre des plateaux (13, 14) pourvus de surfaces différentes pour agir sur le tronçon du tuyau.

7. Dispositif de perfusion suivant la revendication 1, caractérisé en ce que le dispositif de presse (9) est formé par un plateau d'appui (12) sur lequel le tronçon de tuyau prend appui et en ce que de l'autre côté du tuyau, il est prévu un dispositif excentré (10, 11) qui presse le tronçon de tuyau contre le plateau d'appui (12).

8. Dispositif de perfusion suivant la revendication 7, caractérisé en ce que le dispositif excentré est formé par un disque ou galet (10) excentré, sur la périphérie duquel est prévue une bague de glissement (11), qui peut tourner par rapport à l'excentrique (10) et presse le tronçon de tuyau contre le plateau d'appui.

9. Dispositif de perfusion suivant les revendications précédentes, caractérisé en ce que l'entraînement d'au moins un plateau (14) respectivement de l'excentrique (10) se fait à l'intervention d'un électro-aimant.

10. Dispositif de perfusion suivant l'une des revendications 1 à 8, caractérisé en ce que l'entraînement d'au moins un plateau (14) respectivement de l'excentrique (10) a lieu à l'intervention d'un moteur électrique.

11. Dispositif de perfusion suivant l'une des revendications précédentes caractérisé en ce que le tronçon de tuyau en coopération avec le dispositif de presse se compose de caoutchouc de silicone et est relié sans modification de section transversale à un tuyau en PVC du côté aspiration et pression.

12. Dispositif de perfusion suivant l'une des revendications précédentes 1 à 10, caractérisé en ce que le tronçon de tuyau en coopération avec le dispositif de presse (9) présente une autre forme de section transversale et/ou une autre aire de section transversale comparée au reste du tuyau de perfusion.

13. Dispositif de perfusion suivant l'une des revendications 1 à 10 et 12, caractérisé en ce que le tronçon de tuyau en coopération avec le dispositif de presse (9) est un tronçon distinct et relié au tuyau de perfusion proprement dit par une pièce de ramification.

14. Dispositif de perfusion suivant l'une des revendications précédentes, caractérisé en ce qu'il comporte en amont du dispositif de presse (9) une soupape de retenue et/ou un dispositif rétrécissant ou fermant la section de passage du tuyau.

15. Dispositif de perfusion suivant l'une des revendications précédentes, caractérisé en ce qu'il comporte un senseur mesureur (2, 5) qui mesure la vitesse de perfusion et dont le signal de sortie sert à la commande des dispositifs d'actionnement du dispositif d'augmentation de la pression (9).

16. Dispositif de perfusion suivant l'une des revendications 1 à 15, caractérisé en ce que le dispositif de presse est disposé entre un élément obturateur (7) commandé par un dispositif de réglage (6) pour régler la vitesse de perfusion et l'emplacement de perfusion.

17. Dispositif de perfusion suivant l'une des revendications précédentes, caractérisé en ce que la production des fluctuations de pression a lieu périodiquement.

FIG.1

FIG.2

FIG.3

FIG.4

# FIG. 5